# FASCICULE DE BREVET EUROPEEN

(11) **EP 3 259 244 B1**
(45) Date de publication et mention de la délivrance du brevet: **26.12.2018**
(21) Numéro de dépôt: 16705127.5
(22) Date de dépôt: 17.02.2016
(51) Int. Cl.: C07C 31/20, C07C 1/247, C07C 1/36, C07C 11/12, C07C 11/167

(54) **PROCÉDÉ DE PRODUCTION DE DIOLÉFINES À PARTIR D'UNE CHARGE DIOL DILUÉE**
VERFAHREN ZUR HERSTELLUNG VON DIOLEFINEN AUS EINEM VERDÜNNTEN DIOLROHMATERIAL
PROCESS FOR PRODUCING DIOLEFINS FROM A DILUTE DIOL FEEDSTOCK

(30) Priorité: 18.02.2015 FR 1551355
(43) Date de publication de la demande: 27.12.2017
(73) Titulaire: IFP Energies nouvelles, 92500 Rueil-Malmaison (FR); Compagnie Générale des Etablissements Michelin, 63000 Clermont-Ferrand (FR)
(72) Inventeur: RICHARD, Romain, 38070 St Quentin Fallavier (FR); JACQUIN, Marc, 69002 Lyon (FR)
(74) Mandataire: IFP Energies nouvelles
(86) Numéro de dépôt international: PCT/EP2016/053299
(87) Numéro de publication internationale: WO 2016/131844

(56) Documents cités:
- FR-A- 859 902
- US-A- 2 383 205

## Description

### DOMAINE TECHNIQUE DE L'INVENTION

L'invention concerne la production de dioléfines à partir de diols compris dans une charge diluée, et plus particulièrement de diols compris dans un milieu fermentaire. L'invention concerne en particulier la production de 1,3-butadiène.

### ART ANTÉRIEUR

Aujourd'hui, 95% de la production de 1,3-butadiène est assurée par le vapocraquage d'hydrocarbures et l'extraction subséquente des dioléfines au sein d'une coupe de distillation C₄ par des procédés de distillation extractive.

L'évolution du prix des matières premières conduit à opérer les unités de vapocraquage avec des charges de plus en plus légères, car moins coûteuses, entrainant la diminution de production de coupe C₄, et par conséquent de 1,3-butadiène.

D'autres procédés permettent de produire du butadiène à l'échelle industrielle. On peut citer les procédés de déshydrogénation des butènes et des butanes, qui partent d'une ressource hydrocarbures en C₄. On peut aussi mentionner le procédé Lebedev, qui permet d'obtenir du 1,3-butadiène à partir d'éthanol.

Un autre procédé de production de 1,3-butadiène a été opéré à l'échelle pilote dans les années 1945 aux USA, et est décrit par exemple dans les brevets FR 859902, US 2383205, US 2372221, et dans Industrial & Engineering Chemistry, 37 (9), 1945, p.865 à 908. Ce procédé est constitué de trois étapes principales :
- la fermentation de sucre en 2,3-butanediol;
- l'estérification du 2,3-butanediol par un acide carboxylique pour former le diester correspondant;
- la pyrolyse du diester pour produire du 1,3-butadiène et de l'acide carboxylique qui est recyclé à l'étape d'estérification.

Ce procédé est particulièrement intéressant car l'étape de pyrolyse du diester peut être réalisée avec de très bons rendements (typiquement plus de 80%), et le 1,3-butadiène obtenu est de grande pureté (typiquement plus de 99%), ce qui est crucial pour son utilisation dans diverses applications (chimie fine, élastomère).

Différents diols peuvent être obtenus par fermentation. En particulier, la production de 2,3-butanediol à partir de sucre peut être réalisée au stade laboratoire avec *Klebsiella pneumoniae* avec d'excellentes performances, à une concentration finale en 2,3-butanediol dans le moût de fermentation de 160 g.L⁻¹. *Klebsiella oxytoca* a également été utilisée dans des fermentations au stade pilote. Néanmoins, *Klebsiella* est impliquée dans des pathologies pulmonaires graves, ce qui rend son utilisation pour la production de 2,3-butanediol très problématique.

D'autres micro-organismes non pathogènes permettent d'obtenir des diols ayant 4 atomes de carbones. Par exemple, WO 12058508 revendique la production d'éthanol et de 2,3-butanediol par fermentation de gaz de synthèse. WO 10141780 revendique la production de 1,4-butanediol par fermentation de sucre.

Cependant, l'extraction des diols du milieu fermentaire est délicate d'un point de vue opératoire, et très énergivore. En effet, le milieu fermentaire est constitué très majoritairement d'eau, et comprend également des impuretés organiques et de la biomasse. Les températures d'ébullition des diols sont supérieures à celles de l'eau. Par distillation du milieu fermentaire, l'eau est obtenue en tête de colonne, le diol et la biomasse non volatile étant concentrés en fond. Les diols étant des produits visqueux, la séparation entre les diols et la biomasse par filtration entraîne une perte importante de diols. Pour contourner ce problème, les diols peuvent être extraits de la biomasse par extraction à la vapeur d'eau. Mais on récupère alors les diols dilués dans l'eau, qu'il faut à nouveau concentrer.

Un tel procédé est décrit en détail dans l'article de Blom et al. (Recovery of 2,3-butylene glycol from fermentation liquors, Industrial & Engineering Chemistry, 1945, 37 (9), p.865-870). Le milieu fermentaire contient 85% poids d'eau, 4% poids de 2,3-butanediol, 0,2% poids d'acétylméthylcarbinol, 0,5% poids d'éthanol et 8% poids de biomasse. Ce milieu fermentaire est distillé afin d'éliminer les impuretés organiques que sont l'acétylméthylcarbinol et l'éthanol d'une part (éliminés en tête de colonne avec l'eau via la formation d'azéotropes) et concentrer le 2,3-butanediol d'autre part. Par cette opération, on obtient en fond de colonne à distiller une solution aqueuse contenant 14% poids de 2,3-butanediol et 25% poids de biomasse. Cette solution subit ensuite un entraînement à la vapeur, avec un débit massique de vapeur d'eau 6 fois supérieur à celui de l'alimentation, ce qui permet de récupérer en tête une solution aqueuse de diol à 8% poids, dépourvue de biomasse. Enfin, cette solution est distillée afin de récupérer en fond de colonne le 2,3-butanediol avec une pureté de 99%. Lors de cette procédure, de nombreuses difficultés opératoires peuvent survenir, comme le colmatage des contacteurs gaz /liquide, la précipitation de la biomasse ou le moussage de la solution. Le rendement d'extraction du 2,3-butanediol par cette procédure est typiquement de 50%, et peut atteindre dans le meilleur des cas 90%. L'étude réalisée par Blom *et al.* nous montre bien que ce procédé est complexe, peu efficace et énergivore.

D'autres techniques ont été mises en oeuvre afin d'améliorer la séparation du diol du milieu fermentaire, telles que l'extraction liquide-liquide, la séparation par membranes ou l'adsorption. Néanmoins, ces techniques s'avèrent également peu efficaces et/ou difficiles à mettre en oeuvre.

Une alternative bien connue de l'homme du métier consiste à faire réagir le diol en milieu aqueux avec un aldéhyde (ou une cétone) en présence d'un catalyseur acide pour former un acétal (ou un cétal). Cette alternative est particulièrement bien adaptée quand les deux fonctions alcool du diol sont séparées de 2 ou 3 atomes de carbone, comme par exemple avec le 2,3-butanediol ou le 1,3-butanediol, de manière à former un composé hétérocyclique à 5 ou 6 atomes. Cette réaction donne quantitativement l'acétal (ou le cétal), même si le diol du milieu fermentaire est très dilué, la constante d'équilibre étant largement en faveur de la formation des produits.

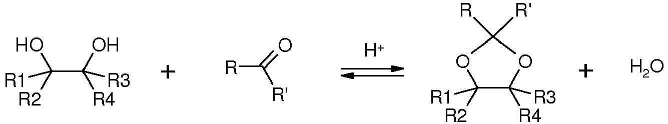

Contrairement au diol dont il est issu, l'acétal (ou le cétal) formé est aprotique et moins polaire, ce qui facilite sa séparation du milieu fermentaire. En effet, l'acétal (ou le cétal) n'est que partiellement soluble dans l'eau et forme donc un azéotrope hétérogène avec l'eau, qui peut être utilisé pour le séparer de la biomasse par distillation. L'acétal (ou le cétal) peut également être extrait du milieu fermentaire par extraction liquide-liquide avec un solvant non miscible dans l'eau (par exemple, un xylène) avec de très bons rendements.

L'extraction du diol en passant par un intermédiaire acétal (ou cétal) semble attractive, mais un inconvénient majeur de cette technique est la procédure de régénération du diol à partir de l'acétal (ou du cétal), qui est aussi complexe et énergivore que l'entraînement à la vapeur décrit précédemment. En effet, elle se déroule en quatre étapes successives :
- une trans-acétalisation (trans-cétalisation) avec un excès de méthanol pour former le diol et un acétal (ou cétal) de méthanol,
- une distillation du mélange méthanol / acétal (ou cétal) de méthanol / diol, avec une première distillation permettant d'obtenir le diol en fond de colonne et un mélange méthanol / acétal (ou cétal) de méthanol en tête, suivi d'une deuxième distillation permettant de séparer le méthanol (qui est recyclé à la première étape) et l'acétal (ou cétal) de méthanol,
- une hydrolyse de l'acétal (ou cétal) de méthanol pour reformer du méthanol et de l'aldéhyde (ou cétone) qui peut être recyclé dans l'étape d'extraction du diol du milieu fermentaire,
- et, enfin, une distillation du mélange méthanol / aldéhyde (ou cétone) / eau, avec une première distillation qui permet de séparer l'aldéhyde (ou la cétone), lequel est recyclé à l'étape de formation de l'acétal de diol, et une deuxième distillation qui permet de séparer l'eau et le méthanol, recyclé à la deuxième étape ci-dessus.

Dans cette procédure, on préfère l'utilisation du méthanol, car ce dernier ne forme pas d'azéotrope avec l'eau, mais tout autre alcool peut être utilisé afin de régénérer le diol à partir de l'acétal (ou cétal).

La présente invention permet de remédier à un ou plusieurs problèmes de l'art antérieur. En effet, la demanderesse a découvert qu'il était possible d'estérifier directement l'acétal avec des performances industriellement intéressantes, ce qui permet de simplifier le procédé de production de dioléfines à partir d'une charge diluée, issue d'une fermentation, comprenant le diol.

### OBJET ET INTÉRÊT DE L'INVENTION

L'invention concerne un procédé de production de dioléfines à partir d'une charge diol diluée, c'est-à-dire contenant de 1 à 30%poids de diol, comprenant au moins les étapes suivantes :
a) une étape d'acétalisation, comprenant une section de réaction-séparation alimentée par ladite charge diol diluée et par une charge carbonyle comprenant au moins l'effluent carbonyle issu de l'étape b) d'estérification, opérée en présence d'un catalyseur acide présent à une concentration comprise entre 1 et 3% molaire par rapport au diol compris dans ladite charge diol diluée, à une température comprise entre 5 et 100°C, le rapport molaire composé carbonylé compris dans ladite charge carbonyle sur diol compris dans ladite charge diol diluée alimentant ladite section de réaction-séparation étant compris entre 1 et 10 et comprenant également au moins une section de séparation de manière à produire au moins un effluent eau et un effluent acétal,
b) une étape d'estérification comprenant une section de réaction-séparation alimentée par l'effluent acétal issu de l'étape a) et comprenant au moins une section de séparation traitant par distillation les effluents de ladite section de réaction-séparation et produisant au moins un effluent eau, un effluent carbonyle, un effluent acide carboxylique concentré et un résidu diester de diol, ladite section de réaction-séparation étant également alimentée par une charge acide carboxylique comprenant au moins ledit effluent acide carboxylique concentré, ladite section de réaction-séparation opérant en présence d'un catalyseur acide, à pression atmosphérique et à une température comprise entre 20°C et 250°C, la quantité de catalyseur mise en oeuvre étant comprise entre 1% et 5% molaire par rapport à la quantité d'acétal introduite, le ratio molaire acide carboxylique / acétal en entrée de ladite section de réaction-séparation étant compris entre 2 et 20,
c) une étape de pyrolyse dudit résidu diester de diol issu de l'étape b) comprenant un réacteur de pyrolyse alimenté par ledit résidu diester de diol, opéré à une température comprise entre 500 et 650°C de manière à produire un effluent de pyrolyse, ladite étape c) comprenant également au moins une section de séparation dans laquelle ledit effluent de pyrolyse est refroidi à une température inférieure à 100°C de manière à produire un effluent de pyrolyse liquide et un effluent de pyrolyse vapeur, et dans laquelle ledit effluent de pyrolyse vapeur est séparé de manière à produire un effluent de composés organiques non condensables et un effluent dioléfines.

Un avantage de l'invention est de pouvoir produire des dioléfines à partir de diols contenus dans une charge diluée avec un nombre d'étapes opératoires réduites par rapport à l'art antérieur, réduisant ainsi la complexité d'opération, la consommation en énergie et le coût. En particulier, il n'est pas nécessaire de régénérer le diol à partir de l'acétal pour obtenir le diester de diol.

### DESCRIPTION DÉTAILLÉE DE L'INVENTION

L'invention concerne un procédé de production de dioléfines à partir d'une charge diol diluée, c'est-à-dire contenant de 1 à 30%poids de diol, comprenant au moins les étapes suivantes :
a) une étape d'acétalisation, comprenant une section de réaction-séparation alimentée par ladite charge diol diluée et par une charge carbonyle comprenant au moins l'effluent carbonyle issu de l'étape b) d'estérification, opérée en présence d'un catalyseur acide présent à une concentration comprise entre 1 et 3% molaire par rapport au diol compris dans ladite charge diol diluée, à une température comprise entre 5 et 100°C, le rapport molaire composé carbonylé compris dans ladite charge carbonyle sur diol compris dans ladite charge diol diluée alimentant ladite section de réaction-séparation étant compris entre 1 et 10 et comprenant également au moins une section de séparation de manière à produire au moins un effluent eau et un effluent acétal,
b) une étape d'estérification comprenant une section de réaction-séparation alimentée par l'effluent acétal issu de l'étape a) et comprenant au moins une section de séparation traitant par distillation les effluents de ladite section de réaction-séparation et produisant au moins un effluent eau, un effluent carbonyle, un effluent acide carboxylique concentré et un résidu diester de diol, ladite section de réaction-séparation étant également alimentée par une charge acide carboxylique comprenant au moins ledit effluent acide carboxylique concentré, ladite section de réaction-séparation opérant en présence d'un catalyseur acide, à pression atmosphérique et à une température comprise entre 20°C et 250°C, la quantité de catalyseur mise en oeuvre étant comprise entre 1% et 5% molaire par rapport à la quantité d'acétal introduite, le ratio molaire acide carboxylique / acétal en entrée de ladite section de réaction-séparation étant compris entre 2 et 20,
c) une étape de pyrolyse dudit résidu diester de diol issu de l'étape b) comprenant un réacteur de pyrolyse alimenté par ledit résidu diester de diol, opéré à une température comprise entre 500 et 650°C de manière à produire un effluent de pyrolyse, ladite étape c) comprenant également au moins une section de séparation dans laquelle ledit effluent de pyrolyse est refroidi à une température inférieure à 100°C de manière à produire un effluent de pyrolyse liquide et un effluent de pyrolyse vapeur, et dans laquelle ledit effluent de pyrolyse vapeur est séparé de manière à produire un effluent de composés organiques non condensables et un effluent dioléfines.

### Charge

Le procédé de production de dioléfines selon l'invention traite une charge diol diluée. Par charge diol diluée, on entend une charge comprenant de 1 à 30% poids de diol, de manière préférée de 10 à 20% poids. Les groupes hydroxyles dudit diol sont préférentiellement liés à deux atomes de carbone séparés par au plus un atome de carbone, comme par exemple dans le cas du 1,3-butanediol. Alternativement et de manière préférée, lesdits groupes hydroxyles sont liés à deux atomes de carbone consécutifs, c'est à dire liés entre eux, comme par exemple dans le cas du 2,3-butanediol. Ledit diol est avantageusement choisi parmi les butanediols, pentanediols et hexanediols, de préférence les butanediols.

Ladite charge diol diluée comprend également de l'eau, de la biomasse et des impuretés organiques. Ladite charge diol diluée comprend avantageusement au moins 50% poids d'eau. Elle comprend avantageusement au moins 5% poids de biomasse. De préférence, ladite charge diol diluée est un milieu fermentaire. Par biomasse, on entend de la matière biologique solide telle que les organismes fermentaires (bactéries, levures), les résidus lignocellulosiques, les résidus végétaux ou animaux non convertis.

### a) Étape d'acétalisation

Par la suite, on utilise le terme acétal pour désigner indifféremment un acétal et un cétal. De même, on utilise le terme acétalisation pour désigner indifféremment la réaction d'acétalisation et de cétalisation. Il est en effet clair pour l'Homme du métier qu'un diol réagit avec un aldéhyde pour produire un acétal et de l'eau par réaction d'acétalisation de la même manière qu'il réagit avec une cétone pour produire un cétal et de l'eau par réaction de cétalisation.

La réaction d'acétalisation est équilibrée, mais en faveur de la formation de l'acétal quand l'acétal est cyclique. Pour consommer la totalité du diol, il est donc nécessaire de mettre en excès le composé carbonylé, et/ou d'extraire l'acétal en couplant la section réactionnelle à une section de séparation. Par composé carbonylé, on entend un aldéhyde ou une cétone, préférentiellement un aldéhyde.

Conformément à l'invention, une étape a) d'acétalisation comprend une section de réaction-séparation alimentée par ladite charge diol diluée et par une charge carbonyle comprenant au moins l'effluent carbonyle issu de l'étape b) d'estérification. Elle comprend également au moins une section de séparation traitant les effluents de ladite section de réaction-séparation et produisant au moins un effluent eau et un effluent acétal.

De préférence, ladite charge carbonyle est constituée de l'effluent carbonyle séparé dans l'étape b) d'estérification et d'un appoint de composé carbonylé permettant de maintenir le ratio molaire composé carbonylé sur diol alimentant ladite section de réaction-séparation.

Le rapport molaire composé carbonylé compris dans ladite charge carbonyle sur diol compris dans ladite charge diol diluée en entrée de ladite section de réaction-séparation est compris entre 1 et 10, de manière préférée entre 1 et 5. Ladite section de réaction-séparation est opérée à pression atmosphérique entre 5°C et 100°C, de manière préférée entre 20°C et 50°C, en présence d'un catalyseur acide homogène, tel que de l'acide sulfurique, de l'acide chlorhydrique, de l'acide paratoluènesulfonique, ou hétérogène, tel qu'une résine échangeuse d'ions avec des fonctionnalités acide sulfonique ou acide carboxylique (par exemple les résines Amberlite, Amberlyst, et Dowex).

Ledit catalyseur acide est présent à une concentration comprise entre 1 et 3% molaire par rapport au diol compris dans ladite charge diol diluée, soit une vitesse molaire horaire (MMH, correspondant au débit molaire de diol divisé par le nombre de moles de catalyseur) comprise entre 20 et 200 h⁻¹.

Ladite section de réaction-séparation peut consister, de manière non limitative, en une distillation réactive ou une extraction liquide-liquide réactive.

Dans un premier mode de réalisation ladite section de réaction-séparation consiste en une extraction liquide-liquide réactive dans laquelle l'acétal formé est extrait sélectivement par un solvant non miscible avec l'eau, choisi parmi un hydrocarbure aliphatique, un hydrocarbure aromatique ou un solvant chloré. Un hydrocarbure aliphatique préféré est le pentane, le n-hexane, le cyclohexane, ou l'éther de pétrole. Un hydrocarbure aromatique préféré est le benzène, le toluène, l'éthylbenzène, les xylènes, la paradiéthylbenzène ou le cumène. Un solvant chloré préféré est le tétrachlorure de carbone. L'eau n'est pas, ou très peu, miscible avec ces solvants. Par ailleurs, le diol éventuellement non converti en acétal a beaucoup plus d'affinité pour la phase aqueuse que pour la phase solvant. En revanche, l'acétal a beaucoup plus d'affinité pour la phase solvant que pour la phase aqueuse.

Dans ce mode de réalisation, ladite charge diol diluée est introduite en tête d'une colonne d'extraction liquide-liquide. Le solvant, non miscible avec l'eau, entre à contre-courant en fond de ladite colonne d'extraction liquide-liquide réactive de manière à extraire l'acétal formé par acétalisation. Selon la nature du composé carbonylé utilisé pour la réaction d'acétalisation, ladite charge carbonyle est introduite en tête de colonne ou en fond de colonne. De manière préférée, le rapport massique des deux phases en présence au sein de la colonne d'extraction liquide-liquide est compris entre 0,2 et 5, de manière préférée entre 0,5 et 2. Le temps de séjour au sein de la colonne d'extraction liquide-liquide, le nombre de plateaux théoriques et le rapport des débits de solvants sont réglés de manière à ce que le rendement d'extraction du diol soit supérieure à 90%, de manière préférée supérieure à 95%. Le rendement d'extraction est égal au ratio molaire du débit de composé acétal dans l'effluent acétal sur le débit de composé diol dans ladite charge diol diluée.

Selon la nature du composé carbonylé utilisé, ce dernier se partage préférentiellement dans la phase aqueuse ou dans la phase solvant. En particulier, plus le nombre d'atomes de carbone du composé carbonyle est faible, plus il sera hydrophile. Dans le cas où le composé carbonylé se partage préférentiellement dans la phase aqueuse, ladite charge carbonyle est introduite en tête de colonne en co-courant avec la charge diol diluée. Dans le cas où le composé carbonylé se partage préférentiellement dans la phase solvant, ladite charge carbonyle est introduite en fond de colonne avec le solvant et circule à contre-courant de la charge diol diluée afin de maximiser les échanges entre les deux phases.

Dans le cas où le composé carbonylé se partage préférentiellement dans la phase aqueuse, il doit avoir une température d'ébullition à pression atmosphérique inférieure à celle de l'eau, préférentiellement inférieure à 80°C, et de manière préférée inférieure à 60°C, afin de rendre aisée sa séparation sans qu'il soit nécessaire de vaporiser l'eau. Par ailleurs, il est préférable que le composé carbonylé ne forme pas d'azéotrope avec l'eau. Pour que le composé carbonylé se partage préférentiellement dans la phase aqueuse, le rapport du nombre total d'atomes de carbone sur le nombre de fonctions carbonyle dudit composé carbonylé doit avantageusement être égal au plus à 3. De manière très avantageuse, ledit composé carbonyle hydrophile peut être choisi parmi le formaldéhyde, l'acétaldéhyde, le propionaldéhyde et l'acétone.

Lorsque le composé carbonylé se partage préférentiellement dans la phase aqueuse, on soutire en tête de ladite colonne d'extraction liquide-liquide un extrait contenant l'acétal formé et le solvant, et en fond un raffinat contenant le composé carbonylé introduit en excès et n'ayant pas réagit et l'eau. Ce raffinat est séparé par distillation en un effluent eau d'une part et en un effluent carbonyle comportant le composé carbonylé d'autre part, ledit effluent carbonyle étant recyclé vers ladite colonne d'extraction liquide-liquide. L'extrait contenant l'acétal formé et le solvant est séparé par distillation en un effluent acétal comprenant plus de 90%, de préférence plus de 95%, de manière très préférée plus de 99% poids d'acétal en tête, et en un effluent solvant en fond, lequel effluent solvant est recyclé vers ladite colonne d'extraction liquide-liquide.

De manière préférée, les couples solvant d'extraction/composé carbonylé de ladite charge carbonyle sont choisis parmi le groupe formé par les couples hexane/formaldéhyde, cyclohexane/formaldéhyde, benzène/formaldéhyde, toluène/formaldéhyde, éthylbenzène/formaldéhyde, tétrachlorure de carbone/formaldéhyde, éthylbenzène/acétaldéhyde, xylènes/acétaldéhyde, cumène/acétaldéhyde, paradiéthylbenzène/acétaldéhyde, éthylbenzène/propionaldéhyde, xylènes/propionaldéhyde, cumène/propionaldéhyde, paradiéthylbenzène/propionaldéhyde, cumène/acétone et paradiéthylbenzène/acétone et de préférence choisi parmi les couples xylènes/acétaldéhyde et paradiéthylbenzène/acétaldéhyde.

Dans le cas où le composé carbonylé se partage préférentiellement dans la phase solvant, il doit être choisi de sorte qu'il soit facilement séparable du solvant et de l'acétal formé. Par facilement, on entend que le composé carbonylé ne forme pas d'azéotropes avec l'acétal et le solvant et que sa température d'ébullition soit relativement différente des autres espèces. Pour que le composé carbonylé se partage préférentiellement dans la phase solvant, le rapport du nombre total d'atomes de carbone sur le nombre de fonctions carbonyle dudit composé carbonylé doit avantageusement être égal au moins à 4. De manière très avantageuse, ledit composé carbonylé peut être choisi parmi la butanone, le butanal, le triméthylacétaldéhyde, le 2-méthylbutanal, le pentanal, l'hexanal et le benzaldéhyde.

De manière générale, on constate que l'acétal formé est moins volatil que le composé carbonylé dont il est issu, que celui-ci soit hydrophile ou hydrophobe.

Lorsque le composé carbonylé se partage préférentiellement dans la phase solvant, on soutire en fond de ladite colonne d'extraction liquide-liquide un raffinat constitué uniquement d'eau, et en tête un extrait qui est un mélange contenant le solvant, l'acétal formé ainsi que le composé carbonylé mis en excès.

Selon un premier mode dudit premier mode de réalisation, lorsque le composé carbonylé a une température d'ébullition inférieure à celle de l'acétal formé, elle-même inférieure à celle du solvant, le composé le plus léger, le composé carbonylé, est séparé grâce à une première colonne de distillation en un effluent carbonyle en tête de colonne, qui est recyclé vers ladite colonne d'extraction liquide-liquide, et un résidu qui est composé de l'acétal formé et du solvant en pied de colonne. Ce résidu est séparé, par exemple par distillation, en un effluent solvant et un effluent acétal comprenant plus de 90%, de préférence plus de 95%, de manière très préférée plus de 99% poids d'acétal. Ledit effluent solvant est recyclé vers ladite colonne d'extraction liquide-liquide.

Selon un second mode dudit premier mode de réalisation, lorsque l'acétal formé est le composé le plus lourd en terme de point d'ébullition (le composé carbonylé étant plus léger ou plus lourd que le solvant), il est possible de séparer le mélange grâce à une seule colonne de distillation en un effluent solvant/carbonyle en tête de colonne et un effluent acétal en pied de colonne comprenant plus de 90%, de préférence plus de 95%, de manière très préférée plus de 99% poids d'acétal. Ledit effluent solvant/carbonyle est recyclé directement vers ladite colonne d'extraction liquide-liquide.

De manière préférée, le composé carbonylé est choisi de sorte que l'acétal formé à partir dudit composé carbonylé est le composé le plus lourd du mélange acétal/carbonyle/solvant, le composé carbonylé pouvant être plus léger ou plus lourd que le solvant d'extraction.

Dans un second mode de réalisation, ladite section de réaction-séparation consiste en une distillation réactive. Dans ce second mode de réalisation, ladite charge carbonyle est hydrophile de sorte que son mélange avec la charge diol diluée est monophasique. Ladite charge carbonyle comprend un composé carbonylé choisi dans le groupe constitué par le formaldéhyde, l'acétaldéhyde, le propionaldéhyde et l'acétone, pris seul ou en mélange. L'acétal formant un azéotrope hétérogène avec l'eau, une colonne de distillation réactive permet de soutirer l'acétal au fur et à mesure de sa formation, déplaçant ainsi l'équilibre vers la formation de l'acétal. Ladite charge diol diluée est introduite en tête d'une colonne de distillation réactive tandis que ladite charge carbonyle est introduite en fond de ladite colonne de distillation réactive. Les débits sont réglés et ladite colonne de distillation réactive est dimensionnée de sorte qu'en fond de colonne on récupère un effluent riche en eau (c'est à dire supérieur à 98% poids d'eau), et qu'en tête de colonne on récupère un mélange d'eau, d'acétal et de composé carbonylé introduit en excès.

Selon un premier mode de ce second mode de réalisation, utilisé de manière préférée quand la proportion du composé carbonylé dans le condensat est faible, et n'engendre donc pas une homogénéisation du mélange liquide eau/composé carbonylé/acétal formé, en tête de colonne, le distillat est refroidi totalement dans un condenseur. Le condensat, constitué d'un mélange eau/composé carbonylé/acétal formé, est envoyé vers un décanteur afin de séparer les deux phases formées (azéotrope hétérogène eau / acétal). Le composé carbonylé se partageant préférentiellement dans la phase aqueuse, on sépare par décantation un flux riche en eau et composé carbonylé qui est renvoyé en tant que reflux vers ladite colonne de distillation réactive et un flux riche en acétal. Ce dernier est séparé par distillation en un effluent acétal comprenant plus de 90%, de préférence plus de 95%, de manière très préférée plus de 99% poids d'acétal en pied de colonne, et un flux azéotrope eau/acétal obtenu en tête de colonne, ce dernier étant recyclé vers ledit décanteur.

Selon un autre mode de ce second mode de réalisation, en particulier quand la proportion de composé carbonylé dans le condensat engendre un système monophasique après condensation totale, en tête de colonne, le distillat est refroidi dans un condenseur partiel réglé de sorte que le composé carbonylé reste sous forme vapeur et constitue l'effluent carbonyle, qui est, après avoir été refroidi, recyclé vers ladite colonne de distillation réactive. Le condensat, constitué d'eau et de l'acétal formé, est envoyé vers un décanteur. Dans une variante de ce mode de l'invention, en tête de colonne, le distillat est refroidi dans un condenseur total, qui est envoyé vers une colonne à distiller permettant de récupérer en tête un effluent carbonyle qui est recyclé en fond de ladite colonne à distiller réactive, et en fond un effluent eau / acétal formé qui est envoyé vers un décanteur. L'eau et l'acétal formant un azéotrope hétérogène, on sépare par décantation un flux riche en eau qui est renvoyé en tant que reflux vers ladite colonne de distillation réactive et un flux riche en acétal. Ce dernier est séparé par distillation en un effluent acétal comprenant plus de 90%, de préférence plus de 95%, de manière très préférée plus de 99% poids d'acétal en pied de colonne, et un flux azéotrope eau/acétal obtenu en tête de colonne, ce dernier étant recyclé vers ledit décanteur.

De préférence, ledit effluent acétal est envoyé directement vers l'étape b) d'estérification, sans aucune étape de régénération du diol à partir de l'acétal.

### b) Étape d'estérification

Conformément à l'invention, une étape b) d'estérification comprend une section de réaction-séparation alimentée par l'effluent acétal issu de l'étape a) et comprend au moins une section de séparation traitant par distillation les effluents de ladite section de réaction-séparation et produisant au moins un effluent eau, un effluent carbonyle, un effluent acide carboxylique concentré et un résidu diester de diol.

Conformément à l'invention, ladite section de réaction-séparation est également alimentée par une charge acide carboxylique comprenant au moins ledit effluent acide carboxylique concentré et comprenant avantageusement un appoint en acide carboxylique de manière à maintenir le ratio acide carboxylique / acétal désiré en entrée de ladite section de réaction-séparation de ladite étape b) d'estérification, c'est à dire un ratio molaire compris entre 2 et 20, préférentiellement entre 4 et 6. De manière préférée, ladite charge acide carboxylique comprend de l'acide acétique.

Au sein de ladite section de réaction-séparation, l'acétal réagit avec l'acide carboxylique pour produire du diester de diol, de l'eau et le composé carbonylé ayant permis de produire l'acétal. Cette réaction est équilibrée, et plutôt en défaveur de la formation du diester de diol. Le composé carbonylé étant volatile, la mise en oeuvre de cette réaction dans une section de réaction-séparation permet de soutirer en continu ledit composé carbonylé produit de la réaction, et de déplacer ainsi l'équilibre de ladite réaction.

Ladite section de réaction-séparation peut être mise en oeuvre, par exemple par distillation réactive. Elle s'opère en présence d'un catalyseur acide homogène, par exemple, l'acide sulfurique, l'acide chlorhydrique, l'acide paratoluènesulfonique ou l'acide benzènesulfonique, ou hétérogène, par exemple une résine acide échangeuse d'ions (de type Amberlyst, Amberlite, Dowex,...), un oxyde mixte (ZrO2, SnO) ou une zéolithe acide (H-MOR, H-MFI, H-FAU et H-BEA), à pression atmosphérique et à une température comprise entre 20°C et 250°C, de manière préférée entre 90°C et 200°C, de manière préférée entre 90°C et 150°C. Préférentiellement, ledit catalyseur acide est hétérogène. La quantité de catalyseur mise en oeuvre est comprise entre 1% et 5% molaire par rapport à la quantité d'acétal introduite, de manière préférée entre 1,5% et 2,5% molaire, soit une vitesse molaire horaire (MMH, correspondant au débit molaire d'acétals divisé par le nombre de moles de catalyseur) comprise entre 0,05 et 25 h⁻¹, préférentiellement entre 0,15 et 20 h⁻¹.

L'effluent de ladite section de réaction-séparation, appelé effluent d'estérification, comprend du diester de diol et des co-produits, parmi lesquels de l'eau, du diol, du mono-ester de diol, de l'acide carboxylique n'ayant pas réagi et le composé carbonylé produit. De manière préférée, ladite section de réaction-séparation est réglée de manière à ce que l'effluent ne contienne plus de diol et de mono-ester de diol, ni d'acétal non converti.

Ladite étape b) d'estérification comprend également au moins une section de séparation dans laquelle ledit effluent d'estérification est ensuite séparé par distillation de manière à obtenir au moins en fond, un résidu diester de diol et en tête, un distillat co-produits comprenant un mélange eau / acide carboxylique / composé carbonylé.

Ledit résidu diester de diol contient plus de 90%, de préférence plus de 95%, de manière très préférée plus de 99% poids de diester de diol. Ledit résidu diester de diol alimente ensuite l'étape c) de pyrolyse.

Ledit distillat co-produits est ensuite séparé, par exemple par distillation, en un effluent carbonyle comprenant le composé carbonylé, qui est recyclé dans le premier réacteur d'acétalisation du diol de ladite étape a) d'acétalisation du procédé selon l'invention, et en un effluent acide carboxylique dilué comprenant un mélange d'eau et d'acide carboxylique.

Ledit effluent acide carboxylique dilué est séparé, typiquement par distillation. Lorsque l'eau et l'acide carboxylique ont une volatilité relative proche de l'unité et ne sont donc pas séparables par simple distillation, ce qui est le cas par exemple de l'eau et de l'acide acétique, on les sépare par distillation azéotropique hétérogène avec comme entraîneur un solvant tel que l'acétate d'éthyle, l'acétate d'isopropyle, le diéthyl éther ou le méthyl tertio-butyl éther. On peut ainsi récupérer d'une part un effluent eau, et d'autre part un effluent acide carboxylique concentré qui peut être recyclé dans ladite section de réaction-séparation de ladite étape b).

### c) Étape de pyrolyse

Conformément à l'invention, une étape c) de pyrolyse comprend un réacteur de pyrolyse alimenté au moins par ledit résidu diester de diol issu de l'étape b) et produisant un effluent de pyrolyse, et comprend une section de séparation séparant ledit effluent de pyrolyse en au moins un effluent de composés organiques non condensables et un effluent dioléfines.

La réaction de pyrolyse transforme le diester de diol en dioléfine et en acide carboxylique. Ledit réacteur de pyrolyse, appelé four de pyrolyse, est opéré à une température comprise entre 500 et 650°C, de préférence entre 550 et 600°C, de préférence entre 575 et 585°C. Le temps de contact optimal au sein du four de pyrolyse est fonction de la pression partielle du diester de diol. Il est typiquement de 1 seconde pour une pression partielle de diester de diol de 1 bar, et de 7 secondes pour une pression partielle de diester de diol de 0,4 bar.

L'effluent issu dudit réacteur de pyrolyse est refroidi rapidement à une température inférieure à 100°C, de préférence inférieure à 50°C, de manière à limiter la formation de composés vinyliques, tels que le vinylcyclohexène dans le cas où la dioléfine est le 1,3-butadiène, par réaction de Diels-Alder des dioléfines sur elles-mêmes. Le refroidissement de l'effluent génère une phase liquide et une phase vapeur qui peuvent être aisément séparées au sein d'un ballon séparateur gaz-liquide en un effluent de pyrolyse liquide et un effluent de pyrolyse vapeur.

Ledit effluent de pyrolyse liquide est composé à plus de 80% poids d'acide carboxylique. Il comprend également d'autres composés organiques comme par exemple le diester de diol non converti, des intermédiaires de pyrolyse tels que, dans le cas où l'acide carboxylique est l'acide acétique et le diol est le 2,3-butanediol, le méthyl vinyl carbinol acétate (MVCA), le méthyl éthyl cétone énol acétate (MEKEA) et le crotyl acétate (CA) et des sous-produits tels que le vinylcyclohexène, la méthyléthylcétone (MEK) ou la méthylacétylacétone (MAA).

Ladite étape c) de pyrolyse comprend également au moins une section de séparation dans laquelle ledit effluent de pyrolyse vapeur est traité. Optionnellement, ladite étape c) de pyrolyse peut comprendre également au moins une section de séparation dans laquelle ledit effluent de pyrolyse liquide est traité.

De préférence, ledit effluent de pyrolyse liquide est traité de manière à séparer l'acide carboxylique des autres composés organiques par un procédé de séparation bien connu de l'homme du métier. Ledit procédé de séparation peut être la distillation (simple, azéotropique homogène ou hétérogène), la distillation extractive, la distillation réactive, l'extraction liquide-liquide, la cristallisation, l'adsorption ou les membranes. Ledit procédé permet d'obtenir un effluent riche en acide carboxylique qui peut être recyclé vers la section de réaction-séparation de ladite étape b) d'estérification en mélange avec la charge acide carboxylique. Ce procédé permet également d'obtenir un effluent composé de diester de diol non converti et des intermédiaires de pyrolyse pouvant conduire à la dioléfine (MVCA et CA dans le cas où l'acide carboxylique est l'acide acétique) qui est recyclé vers le réacteur de pyrolyse de ladite étape c) de pyrolyse afin de maximiser le rendement global en dioléfines. Ce procédé produit également un effluent sous-produits composé des sous-produits tels que le vinylcyclohexène, la MEK, la MEKEA et la MAA dans le cas où l'acide carboxylique est l'acide acétique et le diol est le 2,3-butanediol. Les composés de ce dernier effluent ne pouvant conduire à la dioléfine, ils sont évacués pour être éventuellement valorisés dans un procédé externe.

Ledit effluent de pyrolyse vapeur comprend essentiellement plus de 90%, de préférence plus de 95% poids de dioléfines (sans considérer l'éventuel diluant inerte utilisé pour abaisser la pression partielle de diester de diol au sein du four de pyrolyse). Ledit effluent de pyrolyse vapeur peut également contenir des composés organiques légers, issus de la pyrolyse de l'acide carboxylique, comme par exemple du méthane, du monoxyde de carbone, du dioxyde de carbone, du cétène, de l'hydrogène ou encore de l'éthane. Ledit effluent de pyrolyse vapeur peut-être comprimé et/ou refroidi de manière à condenser les dioléfines. Selon un mode particulier de réalisation, on utilise une colonne et les composés organiques non condensables issus de la pyrolyse de l'acide carboxylique sont ainsi éliminés en tête sous forme d'un effluent de composés organiques non condensables. Les dioléfines, qui sont récupérées en fond de colonne, peuvent ensuite subir une ou plusieurs étapes de purification ultimes bien connues de l'homme du métier. On peut citer de manière non limitative la purification sur tamis ou sur une argile. Ceci permet d'éliminer les impuretés et d'obtenir un effluent dioléfines, lequel comprend plus de 99%, de manière préférée plus de 99,5% de dioléfines.

La conversion du diol compris dans la charge diol diluée, préférentiellement du 2,3-butanediol, en dioléfines, préférentiellement en 1,3-butadiène, par le procédé de pyrolyse selon l'invention est comprise entre 75% et 100%, de préférence entre 75% et 90%.

### Description des figures

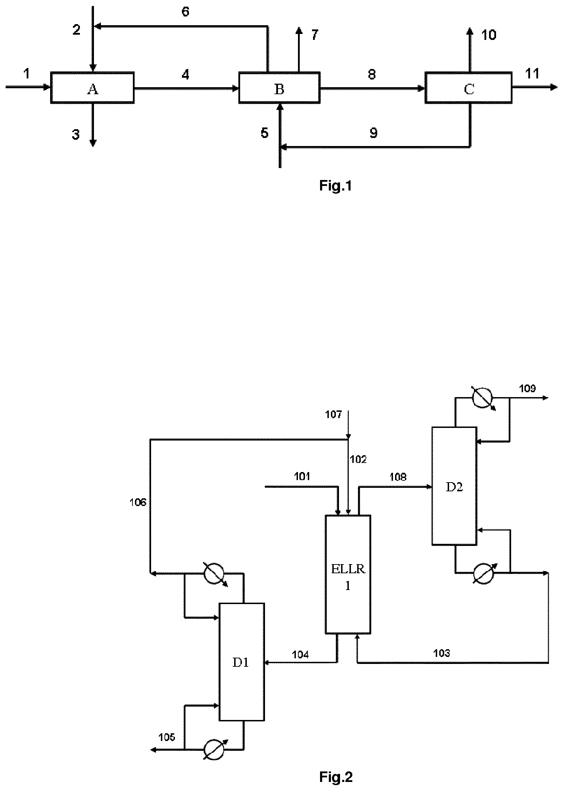

- La figure 1 présente un arrangement particulier du procédé. Une charge diol diluée (1) et une charge carbonyle (2) alimentent une étape (A) d'acétalisation. Cette étape produit au moins un effluent acétal (4) et un effluent eau (3). Ledit effluent acétal (4), ainsi qu'une charge acide carboxylique (5), alimentent, dans une étape (B) d'estérification, une section de réaction-séparation dans laquelle l'acétal est converti en diester de diol. L'effluent de ladite section de réaction-séparation est séparé en un effluent carbonyle (6), qui est recyclé vers l'étape (A) d'acétalisation, un effluent eau (7) et un résidu diester de diol (8). Ce dernier alimente une étape (C) de pyrolyse. Ladite étape (C) de pyrolyse, qui comprend un réacteur de pyrolyse et au moins une section de séparation, produit un effluent riche en acide carboxylique (9) qui est recyclée dans l'étape (B) d'estérification, un effluent de composés organiques non condensables (10) et un effluent dioléfines (11) qui constitue le produit principal du procédé selon l'invention.
- La figure 2 présente un arrangement possible pour l'étape a) d'acétalisation dans laquelle ladite section de réaction-séparation est mise en oeuvre par extraction liquide-liquide réactive avec un composé carbonylé se partageant préférentiellement dans la phase aqueuse. Le solvant utilisé, non miscible à l'eau, a dans ce cas une température d'ébullition plus élevée que celle de l'acétal formé pour ne pas avoir à le vaporiser lors de la séparation.
   La charge diol diluée (*101*) est introduite en tête d'une colonne d'extraction liquide-liquide réactive (*ELLR1*) avec une charge carbonyle (*102*) en excès en co-courant. Un solvant (*103*) non miscible avec l'eau, entre à contre-courant dans la colonne d'extraction liquide-liquide réactive (*ELLR1*) de manière à extraire l'acétal formé par acétalisation. Le raffinat (*104*) est constitué d'eau et du composé carbonylé mis en excès alors que l'extrait (*108*) est un mélange contenant le solvant et l'acétal formé. Le composé carbonylé peut être séparé facilement de l'eau grâce à la colonne de distillation (*D1*). Le composé carbonylé, plus léger, se retrouvent en tête de colonne (*106*) alors que l'eau se retrouve en pied de colonne (*105*). Le flux (*106*) est recyclé dans *ELLR1* et un appoint de composé carbonylé est nécessaire (*107*) car une partie de cette espèce a été consommée pour former l'acétal. Concernant l'extrait (*108*), il est également distillé (*D2*) de sorte que le composé le plus lourd, le solvant dans ce cas, soit obtenu en fond de colonne (*103*) alors que l'acétal se retrouve en tête de colonne (*109*) avant d'être envoyé vers la section de réaction-séparation de l'étape b) d'estérification. Le flux de solvant (*103*) est quant à lui recyclé vers *ELLR1.*

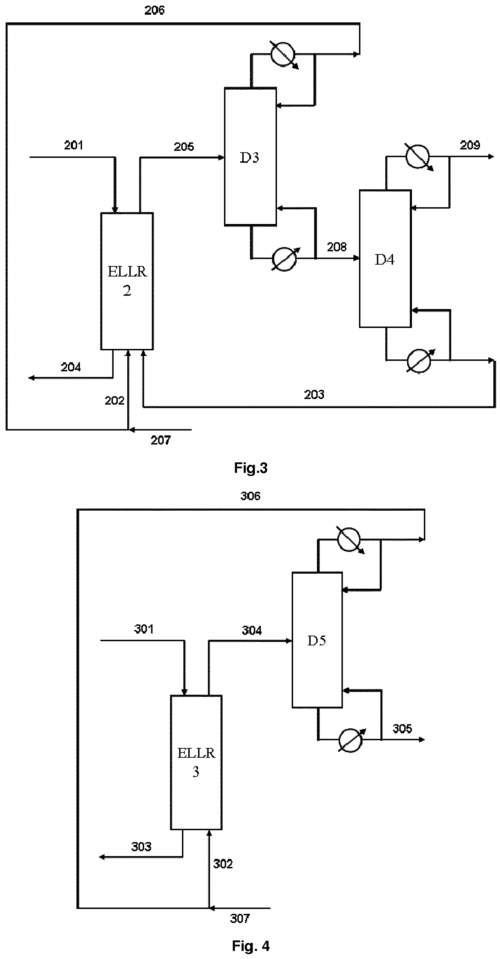

- La figure 3 présente un arrangement possible pour l'étape a) d'acétalisation dans laquelle la section de réaction-séparation est mise en oeuvre par extraction liquide-liquide réactive avec un composé carbonylé se partageant préférentiellement dans la phase solvant. Le solvant utilisé a dans ce cas une température d'ébullition plus élevée que celle de l'acétal formé, elle-même plus élevée que celle du composé carbonylé utilisé.
   La charge diol diluée (*201*) est introduit en tête d'une colonne d'extraction liquide-liquide réactive (*ELLR2*). La charge carbonyle (*202*) en excès est introduite à contre-courant en fond de la colonne d'extraction liquide-liquide réactive (*ELLR2*). Un solvant (*203*) non miscible avec l'eau, entre également à contre-courant dans la colonne d'extraction liquide-liquide réactive (*ELLR2*) de manière à extraire l'acétal formé par acétalisation. Le raffinat (*204*) est constitué uniquement d'eau alors que l'extrait (*205*) est un mélange contenant le solvant, l'acétal formé ainsi que le composé carbonylé mis en excès. Le composé le plus léger, i.e. le composé carbonylé (*206*) dans cet exemple, est séparé grâce à une première colonne de distillation (*D3*). Ce flux (*206*) est recyclé dans *ELLR2* et un appoint de composé carbonylé est nécessaire (*207*) car une partie de cette espèce a été consommée pour former l'acétal. Le pied de la colonne *D3* (*208*) est alors composé de l'acétal et du solvant. Ce flux est aussi distillé (*D4*) de sorte que le composé le plus lourd, le solvant dans cet exemple, soit obtenu en fond de colonne (*203*) alors que l'acétal se retrouve en tête de colonne (*209*) avant d'être envoyé vers la section de réaction-séparation de l'étape b) d'estérification. Le flux de solvant (*203*) est quant à lui recyclé vers *ELLR2.*
- La figure 4 présente un arrangement possible pour l'étape a) d'acétalisation dans laquelle la section de réaction-séparation est mise en oeuvre par extraction liquide-liquide réactive avec un composé carbonylé se partageant préférentiellement dans la phase solvant. L'acétal formé est, dans ce cas, le composé le plus lourd, le composé carbonylé pouvant être plus léger ou plus lourd que le solvant.
   La charge diol diluée (*301*) est introduit en tête d'une colonne d'extraction liquide-liquide réactive (*ELLR3*). La charge carbonyle en excès avec un solvant non miscible à l'eau (*302*) est introduite à contre-courant en fond de la colonne d'extraction liquide-liquide réactive (*ELLR3*). Ce solvant permet d'extraire l'acétal formé par acétalisation. Le raffinat (*303*) est constitué uniquement d'eau alors que l'extrait (*304*) est un mélange contenant le solvant, l'acétal formé ainsi que le composé carbonylé mis en excès. Ce mélange est séparé par une colonne de distillation (*D5*) qui permet d'obtenir, en pied de colonne, un effluent acétal (*305*), et en tête de colonne, un effluent solvant/composé carbonylé (*306*). L'effluent acétal (*305*) est envoyé directement vers la section de réaction-séparation de l'étape b) d'estérification alors que l'effluent solvant/composé carbonylé (*306*) est recyclé vers *ELLR3,* et un appoint de composé carbonylé est nécessaire (*307*) car une partie de cette espèce a été consommée pour former l'acétal.

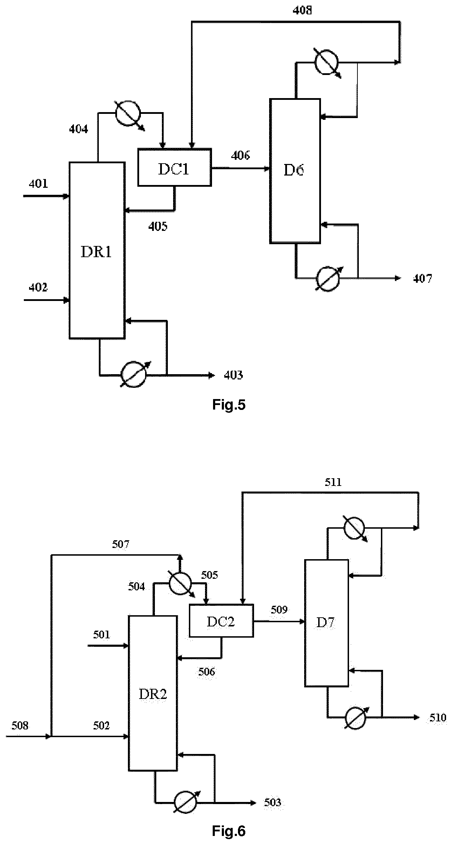

- La figure 5 présente un arrangement possible pour l'étape a) d'acétalisation dans laquelle la section de réaction-séparation est mise en oeuvre par distillation réactive. Dans cet exemple, le distillat est totalement refroidi dans un condenseur, la proportion du composé carbonylé dans le condensat n'engendrant pas une homogénéisation du mélange liquide eau/composé carbonylé/acétal formé.
   La charge diol diluée (*401*) est introduite en tête d'une colonne à distiller réactive (*DR1*), tandis que la charge carbonyle (*402*) est introduite en fond de ladite colonne. On récupère de l'eau en fond de colonne (*403*). En tête de colonne, le distillat (*404*) est totalement refroidi dans un condenseur puis le condensat constitué d'un mélange eau/composé carbonylé/acétal formé, est envoyé vers un décanteur (*DC1*). Le composé carbonylé étant hydrophile, l'eau et l'acétal formant un azéotrope hétérogène, il est aisé de renvoyer un flux riche en eau et composé carbonylé (*405*) vers la colonne *DR1* et de récupérer un flux riche en acétal (*406*). Ce dernier flux est acheminé vers une colonne à distiller (*D6*) permettant d'obtenir de l'acétal pur en pied de colonne (*407*), qui est envoyé vers la section de réaction-séparation de l'étape b) d'estérification, et l'azéotrope eau/acétal obtenu en tête de colonne (*408*) est recyclé vers le décanteur (*DC1*).
- La figure 6 présente un arrangement possible pour l'étape a) d'acétalisation dans laquelle la section de réaction-séparation est mise en oeuvre par distillation réactive. Dans cet exemple, le distillat est refroidi par un condenseur partiel de sorte que le composé carbonylé soit séparé du mélange eau / acétal.
   La charge diol diluée (*501*) est introduite en tête d'une colonne à distiller réactive (*DR1*), tandis que la charge carbonyle (*502*) est introduite en fond de ladite colonne. On récupère de l'eau en fond de colonne (*503*). En tête de colonne, le distillat (*504*) est refroidi dans un condenseur partiel dont la température est réglée de sorte que le composé carbonylé reste sous forme vapeur avant d'être refroidi à son tour pour être recyclé (*507*) vers *DR1.* Un appoint de composé carbonylé est alors nécessaire (*508*) pour maintenir les proportions des réactifs. Le condensat (*505*), constitué d'eau et d'acétal, est quant à lui envoyé vers un décanteur (*DC2*). L'eau et l'acétal formant un azéotrope hétérogène, il est aisé de renvoyer un flux riche en eau vers la colonne (*506*) et de récupérer un flux riche en acétal (*509*). Ce dernier flux est acheminé vers une colonne à distiller (*D7*) permettant d'obtenir de l'acétal pur en pied de colonne (*510*), qui est envoyé vers la section de réaction-séparation de l'étape b) d'estérification, et l'azéotrope eau/acétal obtenu en tête de colonne (*511*) est recyclé vers le décanteur (*DC2*).

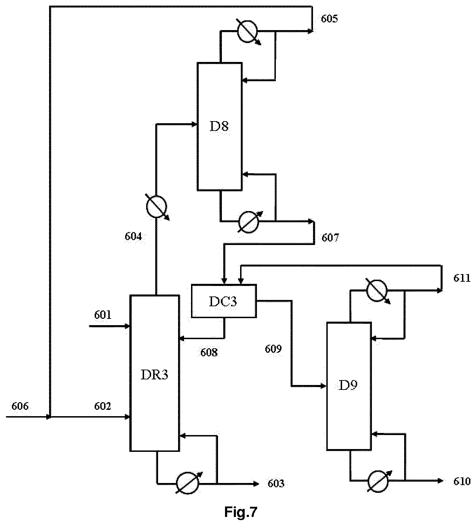

- La figure 7 présente un arrangement possible pour l'étape a) d'acétalisation dans laquelle la section de réaction-séparation est mise en oeuvre par distillation réactive. Dans cet exemple, le distillat est envoyé vers une première colonne de distillation permettant de séparer le composé carbonylé du mélange eau/acétal formé.

La charge diol diluée (*601*) est introduite en tête d'une colonne à distiller réactive (*DR3*), tandis que la charge carbonyle (*602*) est introduite en fond de ladite colonne. On récupère de l'eau en fond de colonne (*603*). En tête de colonne, le distillat (*604*) est refroidi et envoyé vers une colonne de distillation (*D8*). Cette colonne *D8* permet d'obtenir, en tête de colonne, le composé carbonylé (*605*) qui est recyclé vers *DR3,* et en fond de colonne, un mélange eau/acétal formé (*607*) qui est envoyé vers un décanteur *DC3.* Un appoint de composé carbonylé est alors nécessaire (*606*) pour maintenir les proportions des réactifs. L'eau et l'acétal formant un azéotrope hétérogène, il est aisé de renvoyer un flux riche en eau vers la colonne (*608*) et de récupérer un flux riche en acétal (*609*). Ce dernier flux est acheminé vers une colonne à distiller (*D9*) permettant d'obtenir de l'acétal pur en pied de colonne (*610*), qui est envoyé vers la section de réaction-séparation de l'étape b) d'estérification, et l'azéotrope eau/acétal obtenu en tête de colonne (*611*) est recyclé vers le décanteur (*DC3*).

### EXEMPLE

### Exemple 1

Cet exemple illustre la faisabilité de l'estérification directe d'un acétal.

### Acétalisation

Une section de réaction est alimentée par une charge diol diluée comprenant 10% poids de 2,3-butanediol, 6% poids de biomasse constituée de résidus fibreux et d'organismes fermentaires, et 84% poids d'eau et une charge carbonyle constituée d'acétaldéhyde. L'acétaldéhyde est alimenté en excès par rapport au 2,3-butanediol avec un ratio molaire acétaldéhyde / 2,3-butanediol égal à 5.

Cette section de réaction a un volume de 30 mL, maintenu à une température de 40°C et à pression atmosphérique. Elle est opérée en présence d'une résine échangeuse d'ions (Amberlyst 36) présente à une concentration de 2,5% molaire par rapport au 2,3-butanediol présent dans la charge diol diluée. Une conversion du 2,3-butanediol en 2,4,5-triméthyl-1,3-dioxolane par passe de 40% est observée.

Cette section de réaction, couplée à une section de séparation (extraction liquide-liquide réactive) permet d'augmenter la conversion du 2,3-butanediol à 100%.

Les effluents de la section de réaction-séparation sont séparés de manière à obtenir un effluent acétal comprenant 99% poids de 2,4,5-triméthyl-1,3-dioxolane et 1% poids d'eau.

### Estérification

Une section de réaction-séparation de l'étape d'estérification est alimentée par cet effluent acétal ainsi que par une charge acide carboxylique constituée d'acide acétique. L'acide acétique est alimenté en excès par rapport au 2,4,5-triméthyl-1,3-dioxolane, avec un ratio molaire acide acétique / 2,4,5-triméthyl-1,3-dioxolane égal à 6.

Cette section de réaction-séparation a un volume de 30 mL, maintenu à une température de 110°C et à pression atmosphérique, et contenant une résine acide de type Amberlyst 36. La quantité de catalyseur contenue dans la section de réaction-séparation correspond à 2,2% molaire de catalyseur par rapport au 2,4,5-triméthyl-1,3-dioxolane. L'effluent gazeux est refroidi à une température de 20°C.

Le suivi cinétique est assuré par chromatographie en phase gazeuse avec un détecteur à ionisation de flamme et par dosage acido-basique avec de la soude à 0,5 M. Du fait de la température de la section de réaction-séparation, l'acétaldéhyde est séparé en continu lors de la réaction.

L'effluent liquide de la section de réaction-séparation est constitué de 2,1% de 2,3-butanediol, de 30,0% de monoacétate de 2,3-butanediol et de 67,9% de diacétate de 2,3-butanediol.

La conversion directe de l'acétal (le 2,4,5-triméthyl-1,3-dioxolane) en diester de diol (le diacétate de 2,3-butanediol) est bien réalisée en une étape.

L'effluent liquide est ensuite séparé par distillation de manière à obtenir un résidu diester de diol constitué de 97,2% poids de diacétate de 2,3-butanediol et de 2,8% poids d'eau.

### Pyrolyse

Ce résidu diester de diol alimente un four de pyrolyse opéré à 580°C avec un temps de contact d'environs 2 s. L'effluent de pyrolyse est rapidement refroidi à 45°C et se condense en un effluent de pyrolyse liquide. La partie non condensée, qui constitue l'effluent de pyrolyse vapeur, comprend 97,5% poids de 1,3-butadiène.

On obtient donc bien du 1,3-butadiène en mettant en oeuvre une estérification directe d'un acétal et en ne mettant pas en oeuvre d'étapes de régénération du diol à partir de l'acétal telles que décrites dans l'art antérieur.

### Exemple 2

Cet exemple illustre une mise en oeuvre industrielle de l'étape b) d'estérification réalisée par simulation.

### Distillation réactive

La simulation de la colonne de distillation réactive de l'étape b) d'estérification d'un acétal de 2,3-butandiol par l'acide acétique est réalisée à l'aide d'un simulateur de procédé commercial (Aspen). La colonne de distillation réactive simulée est composée de 30 plateaux numérotés de haut en bas. Une première charge (ALIM 1) constituée principalement de 2,4,5-triméthyl-1,3-dioxolane est introduite au niveau du plateau 5. Une seconde charge (ALIM 2) constituée acide acétique est injectée au niveau du plateau 25. La colonne est équipée d'un condenseur partiel liquide-vapeur : une partie du distillat reste sous forme vapeur (DIST-ACH), une autre est condensée et forme une phase liquide (DIST-LIQ). Une partie du distillat liquide (DIST-LIQ) est réinjectée au plateau 1 à titre de reflux. La colonne de distillation réactive produit en fond un résidu (RESIDU), dont une partie est réinjectée au plateau 30 comme reflux du rebouilleur.

Les cinétiques des réactions d'estérification ont été mesurés au sein d'un réacteur batch.

La colonne de distillation réactive est opérée avec les conditions opératoires suivantes :
taux de reflux (molaire) : 1
taux de rebouillage (molaire) : 6,81
pression en tête de colonne : 0,1 MPa
pression en fond de colonne : 0,111 MPa
ratio molaire ALIM 2/ ALIM 1 dans la colonne : 2,73
température en tête de colonne : 40,0°C
température en fond de colonne : 206,5°C
temps de séjour dans la colonne : 2 h
nombre de plateaux réactifs : 7 plateaux répartis uniformément entre ALIM 1 et ALIM 2
temps de séjour par plateau : 17,14 min (on considère que le temps de séjour se répartit équitablement sur tous les plateaux réactifs)

Avec cette configuration et sous ces conditions opératoires, la simulation a conduit aux résultats suivants :

| Débits | ALIM 1 | ALIM 2 | RESIDU | DIST-LIQ | DIST-ACH |
|---|---|---|---|---|---|
| (kmol/hr) | | | | | |
| DIESTER | 0 | 0 | 49,3697 | 2,90E-11 | 1,59E-15 |
| MONOESTER | 0 | 0 | 0,1303 | 6,91E-09 | 6,76E-13 |
| Eau | 0,5 | 0 | 7,81E-12 | 49,4116 | 0,4580 |
| Acide Acétique | 0 | 135 | 2,24E-05 | 36,0199 | 0,1104 |
| DIOL (2,3-butanediol) | 0 | 0 | 2,10E-07 | 6,25E-12 | 1,56E-16 |
| Acétaldéhyde | 0 | 0 | 4,95E-29 | 40,3985 | 9,1015 |
| 2,4,5-triméthyl-1,3-dioxolane | 49,5 | 0 | 2,49E-17 | 2,21E-05 | 3,37E-07 |

| Fraction molaire | | | | | |
|---|---|---|---|---|---|
| DIESTER | 0 | 0 | 0,9974 | 2,30E-13 | 1,64E-16 |
| MONOESTER | 0 | 0 | 0,0026 | 5,49E-11 | 6,99E-14 |
| Eau | 0,01 | 0 | 1,58E-13 | 0,3927 | 0,0474 |
| Acide Acétique | 0 | 1 | 4,53E-07 | 0,2863 | 0,0114 |
| DIOL (2,3-butanediol) | 0 | 0 | 4,24E-09 | 4,97E-14 | 1,62E-17 |
| Acétaldéhyde | 0 | 0 | 1,00E-30 | 0,3211 | 0,9412 |
| 2,4,5-triméthyl-1,3-dioxolane | 0,99 | 0 | 5,02E-19 | 1,75E-07 | 3,48E-08 |
| Total Flow kmol/hr | 50 | 135 | 49,5 | 125,83 | 9,67 |
| Température K | 303,2 | 303,2 | 479,6 | 313,2 | 313,2 |
| Pressure atm | 1,1 | 1,1 | 1,11 | 1 | 1 |

On démontre ainsi qu'avec le procédé selon l'invention, il est possible de transformer un acétal de diol en diester diol avec un très bon rendement (rendement = 99,74%, avec rendement défini comme le débit molaire de diester au résidu sur le débit acétal en entrée) et un d'obtenir un diester d'une grande pureté (P = 99,74%, avec P défini comme le débit molaire de diester au résidu sur le débit molaire total du résidu).

## Revendications

1. Procédé de production de dioléfines à partir d'une charge diol diluée, c'est-à-dire contenant de 1 à 30%poids de diol, comprenant au moins les étapes suivantes :
a) une étape d'acétalisation, comprenant une section de réaction-séparation alimentée par ladite charge diol diluée et par une charge carbonyle comprenant au moins l'effluent carbonyle issu de l'étape b) d'estérification, opérée en présence d'un catalyseur acide présent à une concentration comprise entre 1 et 3% molaire par rapport au diol compris dans ladite charge diol diluée, à une température comprise entre 5 et 100°C, le rapport molaire composé carbonylé compris dans ladite charge carbonyle sur diol compris dans ladite charge diol diluée alimentant ladite section de réaction-séparation étant compris entre 1 et 10 et comprenant également au moins une section de séparation de manière à produire au moins un effluent eau et un effluent acétal,
b) une étape d'estérification comprenant une section de réaction-séparation alimentée par l'effluent acétal issu de l'étape a) et comprenant au moins une section de séparation traitant par distillation les effluents de ladite section de réaction-séparation et produisant au moins un effluent eau, un effluent carbonyle, un effluent acide carboxylique concentré et un résidu diester de diol, ladite section de réaction-séparation étant également alimentée par une charge acide carboxylique comprenant au moins ledit effluent acide carboxylique concentré, ladite section de réaction-séparation opérant en présence d'un catalyseur acide, à pression atmosphérique et à une température comprise entre 20°C et 250°C, la quantité de catalyseur mise en oeuvre étant comprise entre 1% et 5% molaire par rapport à la quantité d'acétal introduite, le ratio molaire acide carboxylique / acétal en entrée de ladite section de réaction-séparation étant compris entre 2 et 20,
c) une étape de pyrolyse dudit résidu diester de diol issu de l'étape b) comprenant un réacteur de pyrolyse alimenté par ledit résidu diester de diol, opéré à une température comprise entre 500 et 650°C de manière à produire un effluent de pyrolyse, ladite étape c) comprenant également au moins une section de séparation dans laquelle ledit effluent de pyrolyse est refroidi à une température inférieure à 100°C de manière à produire un effluent de pyrolyse liquide et un effluent de pyrolyse vapeur, et dans laquelle ledit effluent de pyrolyse vapeur est séparé de manière à produire un effluent de composés organiques non condensables et un effluent dioléfines.

2. Procédé selon la revendication 1 dans lequel ledit effluent acétal issu de l'étape a) est envoyé directement vers l'étape b) d'estérification, sans aucune étape de régénération du diol à partir de l'acétal.

3. Procédé selon l'une des revendications 1 à 2 dans lequel le rapport molaire composé carbonylé compris dans ladite charge carbonyle sur diol compris dans ladite charge diol diluée alimentant ladite section de réaction-séparation de ladite étape a) est compris entre 1 et 5.

4. Procédé selon l'une des revendications 1 à 3 dans lequel ladite section de réaction-séparation de ladite étape a) est une extraction liquide-liquide réactive dans laquelle l'acétal formé est extrait sélectivement par un solvant non miscible avec l'eau.

5. Procédé selon la revendication 4 dans lequel le rapport massique des deux phases en présence au sein de la colonne d'extraction liquide-liquide est compris entre 0,2 et 5.

6. Procédé selon l'une des revendications 4 à 5 dans lequel ledit solvant non miscible avec l'eau est choisi parmi un hydrocarbure aliphatique, un hydrocarbure aromatique ou un solvant chloré.

7. Procédé selon l'une des revendications 4 à 6 dans lequel le rapport du nombre total d'atomes de carbone sur le nombre de fonctions carbonyle dudit composé carbonylé est égal au plus à 3, ladite charge carbonyle étant introduite à co-courant de ladite charge diol diluée.

8. Procédé selon l'une des revendications 4 à 7 dans lequel le rapport du nombre total d'atomes de carbone sur le nombre de fonctions carbonyle dudit composé carbonylé est égal au moins à 4, ladite charge carbonyle étant introduite avec le solvant non miscible avec l'eau à contre-courant de la charge diol diluée.

9. Procédé selon l'une des revendications 1 à 3 dans lequel ladite section de réaction-séparation de ladite étape a) est une distillation réactive alimentée par ladite charge diol dilué en tête de colonne et par ladite charge carbonyle en fond de colonne, ladite charge carbonyle étant hydrophile.

10. Procédé selon la revendication 9 ou la revendication 7 dans lequel ledit composé carbonylé est choisi parmi le formaldéhyde, l'acétaldéhyde, le propionaldéhyde et l'acétone.

11. Procédé selon l'une des revendications 1 à 10 dans lequel ladite section de réaction-séparation de ladite étape b) est opérée à une température comprise entre 90 et 200°C.

12. Procédé selon l'une des revendications 1 à 11 dans lequel ladite section de réaction-séparation de ladite étape b) est opérée à une température comprise entre 90 et 150°C.

13. Procédé selon l'une des revendications 1 à 12 dans lequel le ratio molaire acide carboxylique / acétal en entrée de ladite section de réaction-séparation de ladite étape b) est compris entre 4 et 6.

14. Procédé selon l'une des revendications 1 à 13 dans lequel la quantité de catalyseur mise en oeuvre dans ladite section de réaction-séparation de ladite étape b) est comprise entre 1,5% et 2,5% molaire par rapport à la quantité d'acétal introduite.

15. Procédé selon l'une des revendications 1 à 14 dans lequel l'effluent de pyrolyse est refroidi à une température inférieure à 50°C.

## Patentansprüche

1. Verfahren zur Herstellung von Diolefinen aus einer verdünnten Diol-Charge, d.h. enthaltend 1 bis 30 Gew.-% Diol, umfassend mindestens die folgenden Schritte:
a) einen Acetalisierungsschritt, umfassend eine Sektion zur Reaktion-Trennung, die mit der verdünnten Diol-Charge und einer Carbonyl-Charge versorgt wird, umfassend mindestens einen Carbonyl-Abstrom aus dem Veresterungsschritt b), welcher betrieben wird in Anwesenheit eines sauren Katalysators, der eine Konzentration zwischen 1 und 3 Mol-%, bezogen auf das Diol in der verdünnten Diol-Charge, aufweist, bei einer Temperatur zwischen 5 und 100°C, wobei das Molverhältnis der Carbonylverbindung, die in der Carbonyl-Charge enthalten ist, zu dem Diol, das in der verdünnten Diol-Charge enthalten ist, welche die Sektion zur Reaktion-Trennung versorgt, zwischen 1 und 10 liegt, und auch umfassend mindestens eine Sektion zur Trennung, um mindestens einen Wasserabstrom und einen Acetalabstrom zu erzeugen,
b) einen Veresterungsschritt, umfassend eine Sektion zur Reaktion-Trennung, die mit dem Acetalabstrom von Schritt a) versorgt wird, und umfassend mindestens eine Sektion zur Trennung, die durch Destillation die Abströme der Sektion zur Reaktion-Trennung behandelt und mindestens einen Wasserabstrom, einen Carbonylabstrom, einen konzentrierten Carbonsäureabstrom und einen Dioldiesterrückstand erzeugt, wobei die Sektion zur Reaktion-Trennung auch von einer Carbonsäure-Charge versorgt wird, umfassend mindestens den konzentrierten Carbonsäureabstrom, wobei die Sektion zur Reaktion-Trennung in Anwesenheit eines sauren Katalysators bei Atmosphärendruck und bei einer Temperatur zwischen 20°C und 250°C betrieben wird, wobei die verwendete Katalysatormenge zwischen 1 Mol-% und 5 Mol-%, bezogen auf die eingebrachte Acetalmenge, beträgt, wobei das Molverhältnis von Carbonsäure/Acetal am Eingang der Sektion zur Reaktion-Trennung zwischen 2 und 20 beträgt,
c) einen Pyrolyseschritt des Dioldiesterrückstands von Schritt b), umfassend einen Pyrolysereaktor, der mit dem Dioldiesterrückstand versorgt wird, und bei einer Temperatur zwischen 500 und 650°C betrieben wird, um einen Pyrolyseabstrom zu erzeugen, wobei der Schritt c) auch mindestens einen Sektion zur Trennung umfasst, in welcher der flüssige Pyrolyseabstrom auf eine Temperatur von weniger als 100°C abgekühlt wird, um einen flüssigen Pyrolyseabstrom und einen Dampfpyrolyseabstrom zu erzeugen, und in welcher der Dampfpyrolyseabstrom abgetrennt wird, um einen Abstrom nicht-kondensierbarer organischer Verbindungen und einen Diolefinabstrom zu erzeugen.

2. Verfahren nach Anspruch 1, wobei der Acetalabstrom von Schritt a) direkt zum Veresterungsschritt b) ohne jeden Regenerierungsschritt des Diols aus dem Acetal geführt wird.

3. Verfahren nach einem der Ansprüche 1 bis 2, wobei das Molverhältnis der Carbonylverbindung, die in der Carbonyl-Charge enthalten ist, zu dem Diol, das in der verdünnten Diol-Charge enthalten ist, welche die Sektion zur Reaktion-Trennung von Schritt a) versorgt, zwischen 1 und 5 beträgt.

4. Verfahren nach einem der Ansprüche 1 bis 3, wobei die Sektion zur Reaktion-Trennung von Schritt a) eine reaktive Flüssig-Flüssig-Extraktion ist, bei welcher der gebildete Acetal selektiv durch ein mit Wasser nicht mischbares Lösungsmittel extrahiert wird.

5. Verfahren nach Anspruch 4, wobei das Massenverhältnis der beiden Phasen im Inneren der Flüssig-Flüssig-Extraktionssäule zwischen 0,2 und 5 beträgt.

6. Verfahren nach einem der Ansprüche 4 bis 5, wobei das mit Wasser nicht mischbare Lösungsmittel aus einem aliphatischen Kohlenwasserstoff, einem aromatischen Kohlenwasserstoff oder einem chlorierten Lösungsmittel ausgewählt wird.

7. Verfahren nach einem der Ansprüche 4 bis 6, wobei das Verhältnis der Gesamtanzahl von Kohlenstoffatomen zur Anzahl von Carbonylfunktionen der Carbonylverbindung höchstens gleich 3 ist, wobei die Carbonyl-Charge im Gleichstrom mit der verdünnten Diol-Charge eingebracht wird.

8. Verfahren nach einem der Ansprüche 4 bis 7, wobei das Verhältnis der Gesamtanzahl von Kohlenstoffatomen zur Anzahl von Carbonylfunktionen der Carbonylverbindung mindestens gleich 4 ist, wobei die Carbonyl-Charge mit dem mit Wasser nicht mischbaren Lösungsmittel im Gegenstrom mit der verdünnten Diol-Charge eingebracht wird.

9. Verfahren nach einem der Ansprüche 1 bis 3, wobei die Sektion zur Reaktion-Trennung von Schritt a) eine reaktive Destillation ist, die mit der verdünnten Diol-Charge am Kopf der Säule und mit der Carbonyl-Charge am Boden der Säule versorgt wird, wobei die Carbonyl-Charge hydrophil ist.

10. Verfahren nach Anspruch 9 oder Anspruch 7, wobei die Carbonylverbindung aus Formaldehyd, Acetaldehyd, Propionaldehyd und Aceton ausgewählt wird.

11. Verfahren nach einem der Ansprüche 1 bis 10, wobei die Sektion zur Reaktion-Trennung von Schritt b) bei einer Temperatur zwischen 90 und 200°C betrieben wird.

12. Verfahren nach einem der Ansprüche 1 bis 11, wobei die Sektion zur Reaktion-Trennung von Schritt b) bei einer Temperatur zwischen 90 und 150°C betrieben wird.

13. Verfahren nach einem der Ansprüche 1 bis 12, wobei das Molverhältnis von Carbonsäure/Acetal am Eingang der Sektion zur Reaktion-Trennung von Schritt b) zwischen 4 und 6 liegt.

14. Verfahren nach einem der Ansprüche 1 bis 13, wobei die verwendete Katalysatormenge in der Sektion zur Reaktion-Trennung von Schritt b) zwischen 1,5 Mol-% und 2,5 Mol-%, bezogen auf die eingebrachte Acetalmenge, beträgt.

15. Verfahren nach einem der Ansprüche 1 bis 14, wobei der Pyrolyseabstrom auf eine Temperatur von weniger als 50°C abgekühlt wird.

## Claims

1. Process for producing diolefins from a dilute diol feedstock, i.e. a feedstock containing from 1% to 30% by weight of diol, comprising at least the following steps:
a) an acetalization step, comprising a reaction-separation section fed with said dilute diol feedstock and with a carbonyl feedstock comprising at least the carbonyl effluent resulting from the esterification step b), carried out in the presence of an acid catalyst present at a concentration of between 1 and 3 mol% relative to the diol included in said dilute diol feedstock, at a temperature of between 5°C 100°C, the molar ratio of the carbonyl compound included in said carbonyl feedstock to the diol included in said dilute diol feedstock feeding said reaction-separation section being between 1 and 10 and also comprising at least one separation section so as to produce at least one water effluent and one acetal effluent,
b) an esterification step comprising a reaction-separation section fed with the acetal effluent resulting from step a) and comprising at least one separation section treating, by distillation, the effluents of said reaction-separation section and producing at least one water effluent, one carbonyl effluent, one concentrated carboxylic acid effluent and one diol diester residue, said reaction-separation section also being fed with a carboxylic acid feedstock comprising at least said concentrated carboxylic acid effluent, said reaction-separation section operating in the presence of an acid catalyst, at atmospheric pressure and at a temperature of between 20°C and 250°C, the amount of catalyst used being between 1 and 5 mol% relative to the amount of acetal introduced, the carboxylic acid/acetal molar ratio at the inlet of said reaction-separation section being between 2 and 20,
c) a step of pyrolysis of said diol diester residue resulting from step b) comprising a pyrolysis reactor fed with said diol diester residue, operated at a temperature of between 500°C and 650°C so as to produce a pyrolysis effluent, said step c) also comprising at least one separation section in which said pyrolysis effluent is cooled to a temperature below 100°C so as to produce a liquid pyrolysis effluent and a vapor pyrolysis effluent, and in which said vapor pyrolysis effluent is separated so as to produce an effluent of non-condensable organic compounds and a diolefin effluent.

2. Process according to Claim 1, in which said acetal effluent resulting from step a) is sent directly to the esterification step b), without any step of regenerating the diol from the acetal.

3. Process according to either of Claims 1 and 2, in which the molar ratio of the carbonyl compound included in said carbonyl feedstock to the diol included in said dilute diol feedstock feeding said reaction-separation section of said step a) is between 1 and 5.

4. Process according to one of Claims 1 to 3, in which said reaction-separation section of said step a) is a reactive liquid-liquid extraction in which the acetal formed is selectively extracted by a water-immiscible solvent.

5. Process according to Claim 4, in which the molar ratio of the two phases present within the liquid-liquid extraction column is between 0.2 and 5.

6. Process according to either of Claims 4 and 5, in which said water-immiscible solvent is chosen from an aliphatic hydrocarbon, an aromatic hydrocarbon or a chlorinated solvent.

7. Process according to one of Claims 4 to 6, in which the ratio of the total number of carbon atoms to the number of carbonyl functions of said carbonyl compound is equal at most to 3, said carbonyl feedstock being introduced co-currently with said dilute diol feedstock.

8. Process according to one of Claims 4 to 7, in which the ratio of the total number of carbon atoms to the number of carbonyl functions of said carbonyl compound is equal at least to 4, said carbonyl feedstock being introduced with the water-immiscible solvent counter-currently to the dilute diol feedstock.

9. Process according to one of Claims 1 to 3, in which said reaction-separation section of said step a) is a reactive distillation fed with said dilute diol feedstock at the top of the column and with said carbonyl feedstock at the bottom of the column, said carbonyl feedstock being hydrophilic.

10. Process according to Claim 9 or Claim 7, in which said carbonyl compound is chosen from formaldehyde, acetaldehyde, propionaldehyde and acetone.

11. Process according to one of Claims 1 to 10, in which said reaction-separation section of said step b) is operated at a temperature of between 90°C and 200°C.

12. Process according to one of Claims 1 to 11, in which said reaction-separation section of said step b) is operated at a temperature of between 90°C and 150°C.

13. Process according to one of Claims 1 to 12, in which the carboxylic acids/acetal molar ratio at the inlet of said reaction-separation section of said step b) is between 4 and 6.

14. Process according to one of Claims 1 to 13, in which the amount of catalyst used in said reaction-separation section of said step b) is between 1.5 and 2.5 mol% relative to the amount of acetal introduced.

15. Process according to one of Claims 1 to 14, in which the pyrolysis effluent is cooled to a temperature below 50°C.
